# EUROPEAN PATENT APPLICATION

(11) **EP 4 053 267 A1**
(43) Date of publication of application: **07.09.2022**
(21) Application number: 20882075.3
(22) Date of filing: 30.10.2020
(51) Int. Cl.: C12N 5/0783, C12N 5/10

(54) **METHOD FOR PRODUCING T CELL**

(30) Priority: 01.11.2019 JP 2019199781
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO Shin, Kyoto-shi Kyoto 606-8501 (JP); YANO Hisashi, Kyoto-shi Kyoto 606-8501 (JP); SHINOHARA Tokuyuki, Fujisawa-shi Kanagawa 251-0012 (JP)
(74) Representative: Jones, Nicholas Andrew
(86) International application number: PCT/JP2020/040732
(87) International publication number: WO 2021/085576

(57) **Abstract**

Provided is an excellent method for producing an IL-4 non-secreting and IFN-γ secreting (Th1-type) or IFN-γ non-secreting and IL-4 secreting (Th2-type) CD4 single-positive T cell (CD4SP T cell). The method for producing the Th1-type or Th2-type CD4SP T cell of the present invention comprises a step of inducing a CD4 single-positive T cell from a hematopoietic stem cell (HSC) and/or a hematopoietic progenitor cell (HPC) substantially defective in a factor involved in IL-4 secretion or a factor involved in IFN-γ secretion.

SEQUENCE LISTING

<110>  Kyoto University
       Takeda Pharmaceutical Company Limited

<120>  Method for Producing T cells

<130>  PT38-9050

<150>  JP2019-199781
<151>  2019-11-01

<160>  3

<170>  PatentIn version 3.5

<210>  1
<211>  20
<212>  DNA
<213>  Artificial Sequence

<220>
<223>  Target sequence in IL4 gene for gRNA4

<400>  1
gtgtccgtgg acaaagttgc                                                   20


<210>  2
<211>  20
<212>  DNA
<213>  Artificial Sequence

<220>
<223>  Target sequence in IL4 gene for gRNA5

<400>  2
caagtgcgat atcaccttac                                                   20


<210>  3
<211>  20
<212>  DNA
<213>  Artificial Sequence

<220>
<223>  Target sequence in TBX21 gene for gRNA4

<400>  3
gcggtaccag agcggcaagt                                                   20

## Description

### BACKGROUND OF THE INVENTION

### Field of the Invention

The present invention relates to a method for producing an IL-4 non-secreting and IFN-γ secreting (Th1-type) CD4 single-positive T cell and an IFN-γ non-secreting and IL-4 secreting (Th2-type) CD4 single-positive T cell, similar to a natural Th1 cell and Th2 cell, for example, using an artificial thymic organoid (ATO) comprising a hematopoietic stem cell (HSC) and/or a hematopoietic progenitor cell (HPC) differentiated from an iPS cell. The present invention also relates to a method for controlling differentiation of a hematopoietic stem cell (HSC) and/or a hematopoietic progenitor cell (HPC) into a Th1-type and/or Th2-type CD4 single-positive T cell.

### Background of the Invention

The differentiation of cytotoxic T cells, that is, CD8 single-positive T cells (sometimes referred to as "CD8SP T cells" in the present specification) from iPS cells, the introduction of antigen-specific TCRs (T cell receptors) or CARs (chimeric antigen receptors), and their application to tumor immunotherapy have been the subjects of active research. Such tumor immunotherapy has been developed by focusing on the excellent antitumor activity of cytotoxic T cells. However, the reason behind this is that there was no technology to induce helper T cells, that is, CD4 single-positive T cells (sometimes referred to as "CD4SP T cells" in the present specification) from iPS cells. If helper T cells could be induced, it could be applied to enhance both cell-mediated and humoral immunity, and enhance the effect of immune cell therapy. For example, in CAR-T therapy, administering CD4SP T cells together with CAR-introduced CD8SP T cells is known to enhance the therapeutic effect. In addition, if Th1 cells and Th2 cells, which are subsets of helper T cells, can be specifically produced or amplified, other applications than tumor immunotherapy, for example, controlling the Th1/2 balance which is the pathological background of allergic diseases, becomes possible. If they can be further induced to suppressive T cells (Treg cells), it may be possible to treat allergic or autoimmune diseases by suppressing cell-mediated/humoral immunity. The ability to prepare Th1 cells, Th2 cells, Treg cells, and the like from iPS cells is important for enhancing the effect of immune cell therapy and expanding the variation.

As a method for producing CD8SP T cells and/or CD4SP T cells, a method including a step of culturing three-dimensional cell aggregates containing stromal cells expressing a Notch ligand, hematopoietic progenitor cells and the like, that is, artificial thymic organoids (ATOs), as described in Patent Literature 1, is known. In the Examples of Patent Literature 1, as a more specific embodiment of the method, it is disclosed that ATOs were prepared using (primary) human hematopoietic stem/progenitor cells (HSPCs) isolated from cord blood or the like, that CD8SP T cells, CD4SP T cells, and CD4/CD8 double positive T cells (sometimes referred to as "CD4/CD8DP T cells" in the present specification) were obtained from the ATOs, and that treating the CD4SP T cells with PMA/ionomycin allowed to obtain a very small number of cells expressing only IFN-γ and cells expressing only IL-4 (Figures 4B, 6B, etc. of Patent Literature 1).

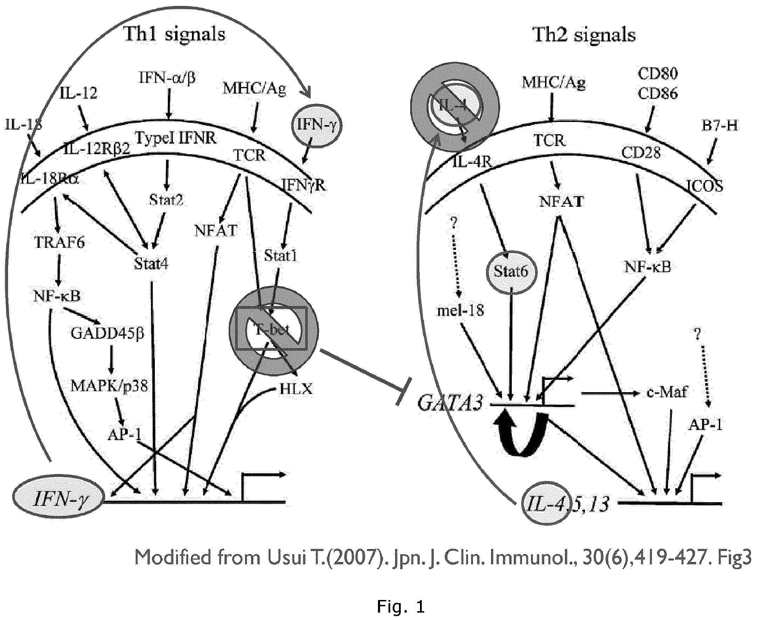

Non Patent Literature 1 describes that hematopoietic stem/progenitor cells (CD34⁺CD3⁻HSPC) collected from cord blood, bone marrow, peripheral blood, or the like were co-cultured with stromal cells expressing a Notch ligand (MS5-hDLL1 cell line) to prepare ATOs, and that CD8SP T cells and CD4SP T cells were produced from the ATOs in addition to CD4/CD8DP T cells (Figure 1b, 2b, 3b, 4a, 4b, and 4f).

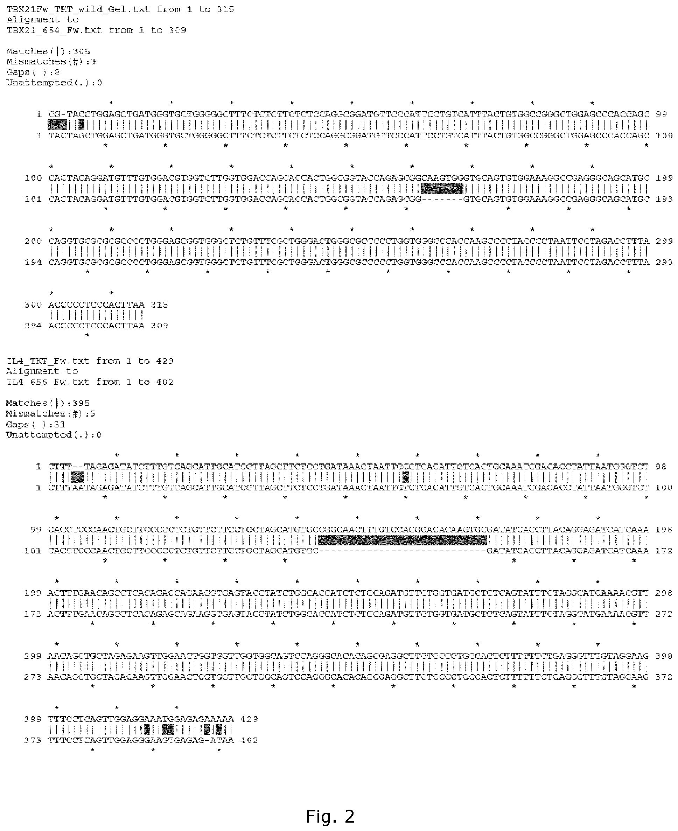

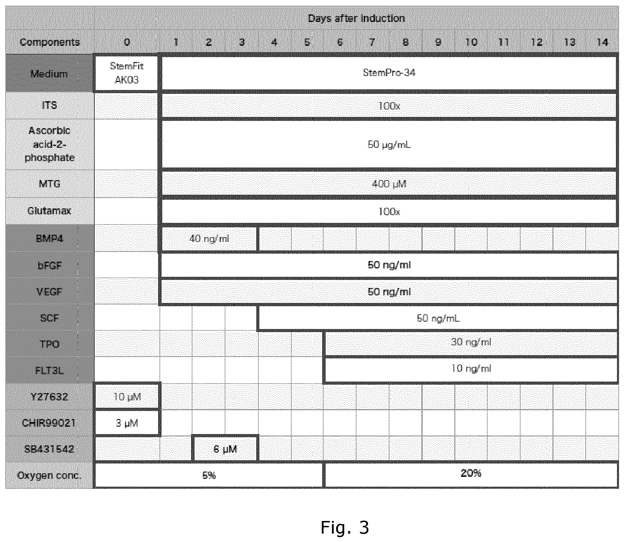

Non Patent Literature 2 describes that embryonic mesodermal progenitors (EMPs) differentiated from human iPS cells were co-cultured with stromal cells expressing a Notch ligand (MS5-hDLL1/4 cell line) to prepare embryonic mesodermal organoids (EMOs),then further induced into a hematopoietic lineage to prepare ATOs, that CD8SP (CD8αβ⁺) T cells and a small number of CD4SP (CD4⁺) T cells were produced from the ATOs in addition to CD4/CD8DP T cells (Figure 2C), and that cells expressing IFN-γ and/or IL-2 were obtained by treating the CD8SP T cells with PMA/ionomycin (Figure 3F). It is also described that while mature naive T cells are obtained and TCRs have a various repertoire when the human iPS cells are prepared from fibroblasts, when iPS cells are modified to express a tumor-associated antigen (NY-ESO-1 peptide)-specific TCR, T cells expressing such TCR are obtained.

However, none of the above three literatures describes a method capable of efficiently preparing IFN-γ secreting (Th1-type) CD4SP T cells and IL-4 secreting (Th2-type) CD4SP T cells, or a method for controlling the differentiation of hematopoietic stem cells (HSCs) and/or hematopoietic progenitor cells (HPCs) into Th1-type and/or Th2-type CD4 single-positive T cells.

Non Patent Literature 3 describes a transcription factor involved in Th1 differentiation (INF-γ gene expression) and its signal transduction, and a transcription factor involved in Th2 differentiation (IL-4 gene expression) and its signal transduction.

### Citation List

### Patent Literature

Patent Literature 1: WO2017/075389

### Non Patent Literature

Non Patent Literature 1: Seet et al., Nature Methods vol.14 no.5, p521-530 (2017)
Non Patent Literature 2: Montel-Hagen et al., Cell Stem Cell. 2019 Mar 7;24(3):376-389
Non Patent Literature 3: Usui., Jpn.J.Clin.Immunol., 30(6), p419-427 (2007)

### SUMMARY OF THE INVENTION

### Technical Problem

As described above, while there is a need for preparing IFN-γ secreting (Th1-type) CD4SP T cells and IL-4 secreting (Th2-type) CD4SP T cells from iPS cells, the means to realize this have not yet been achieved. In particular, when taking into consideration the application and production efficiency in immune cell therapy, it is extremely useful to be able to use hematopoietic stem/progenitor cells differentiated from iPS cells or established iPS cell lines prepared from cells collected from a patient, or iPS cells that have been further modified, rather than primary hematopoietic stem/progenitor cells, in order to prepare ATOs for producing CD4SP T cells as described above.

It should be noted that the primary T cells collected from a living body (peripheral blood), not the T cells produced from ATOs, are specific to secrete either IFN-γ or IL-4 when stimulated with PMA/ionomycin. Therefore, Th1 cells or Th2 cells can be obtained by using primary T cells. By contrast, in the case of ATO prepared using hematopoietic progenitor cells (HPC) induced from iPS cells, when the T cells generated from the ATO are stimulated with PMA/ionomycin, they do not have sufficient specificity for the secretion of IFN-γ and IL-4, and it is not possible to selectively obtain Th1-type or Th2-type CD4SP T cells secreting either one.

An object of the present invention is to provide an excellent method for producing an IL-4 non-secreting and IFN-γ secreting (Th1-type) or IFN-γ non-secreting and IL-4 secreting (Th2-type) CD4 single-positive T cell (CD4SP T cell). Another object of the present invention is to provide a method for controlling differentiation into Th1-type or Th2-type CD4 single-positive T cell.

### Solution to Problem

The present inventors have found that Th1-type CD4SP T cells and Th2-type CD4SP T cells can be efficiently produced by culturing hematopoietic progenitor cells or hematopoietic stem cells substantially defective in a factor involved in IL-4 secretion or a factor involved in IFN-γ secretion, respectively, for example, by preparing ATOs using hematopoietic progenitor cells obtained from iPS cells in which IL4 gene or TBX21 gene is knocked out, and culturing the ATOs.

It should be noted that high secretion of IL-4 and high expression of T-bet were observed in T cell redifferentiation of wild-type iPS cells, and therefore it was also considered that the differentiation itself into mature helper T cells may be inhibited by knocking out the IL4 gene and TBX21 gene encoding them. However, it is a surprising finding that knocking out the IL4 gene and the TBX21 gene did not cause such inhibition, and that Th1-type and Th2-type CD4SP T cells could be prepared.

That is, the present invention includes at least the following:
[1] A method for producing a Th1-type or Th2-type CD4 single-positive T cell, comprising
   a step of inducing a CD4 single-positive T cell from a hematopoietic stem cell (HSC) and/or a hematopoietic progenitor cell (HPC) substantially defective in
   a factor involved in IL-4 secretion, or
   a factor involved in IFN-γ secretion.
[2] The method according to [1] which is a method for producing a Th1-type CD4 single-positive T cell, comprising
   a step of inducing a CD4 single-positive T cell from (B1) a hematopoietic stem cell (HSC) and/or a hematopoietic progenitor cell (HPC) substantially defective in a factor involved in IL-4 secretion.
[3] The method according to [1] which is a method for producing a Th2-type CD4 single-positive T cell, comprising
   a step of inducing a CD4 single-positive T cell from (B2) a hematopoietic stem cell (HSC) and/or a hematopoietic progenitor cell (HPC) substantially defective in a factor involved in IFN-γ secretion.
[4] The method according to [1], wherein the HSC and/or HPC is (b1) a HSC and/or a HPC in which a gene involved in IL-4 secretion is knocked out.
[5] The method according to [1], wherein the HSC and/or HPC is (b2) a HSC and/or a HPC in which a gene involved in IFN-γ secretion is knocked out.
[6] The method according to [1] which is a method for producing a Th1-type CD4 single-positive T cell, comprising
   a step of culturing an artificial thymic organoid (ATO) comprising:
      (a) a stromal cell expressing a Notch ligand; and
      (b1) a hematopoietic stem cell (HSC) and/or a hematopoietic progenitor cell (HPC) in which a gene involved in IL-4 secretion is knocked out, but a gene involved in IFN-γ secretion is not knocked out,
   and thereby producing an IL-4 non-secreting and IFN-γ secreting (Th1-type) CD4 single-positive T cell from the ATO.

   [6-1] A method for producing a Th1-type CD4 single-positive T cell, comprising
      a step of culturing an artificial thymic organoid (ATO) comprising:
         (a) a stromal cell expressing a Notch ligand; and
         (b1) a hematopoietic stem cell (HSC) or a hematopoietic progenitor cell (HPC) in which a gene involved in IL-4 secretion is knocked out, but a gene involved in IFN-γ secretion is not knocked out,
      thereby producing an IL-4 non-secreting and IFN-γ secreting (Th1-type) CD4 single-positive T cell from the ATO.
   [6a] The method according to [6] or [6-1], wherein the gene involved in IL-4 secretion is IL4 gene.
[7] The method according to [1] which is a method for producing a Th2-type CD4 single-positive T cell, comprising
   a step of culturing an artificial thymic organoid (ATO) comprising:
      (a) a stromal cell expressing a Notch ligand; and
      (b2) a hematopoietic stem cell (HSC) and/or a hematopoietic progenitor cell (HPC) in which a gene involved in IFN-γ secretion is knocked out, but a gene involved in IL-4 secretion is not knocked out,
   and thereby producing an IFN-γ non-secreting and IL-4 secreting (Th2-type) CD4 single-positive T cell from the ATO.

   [7-1] A method for producing a Th2-type CD4 single-positive T cell, comprising
      a step of culturing an artificial thymic organoid (ATO) comprising:
         (a) a stromal cell expressing a Notch ligand; and
         (b2) a hematopoietic stem cell (HSC) or a hematopoietic progenitor cell (HPC) in which a gene involved in IFN-γ secretion is knocked out, but a gene involved in IL-4 secretion is not knocked out,
         thereby producing an IFN-γ non-secreting and IL-4 secreting (Th2-type) CD4 single-positive T cell from the ATO.
   [7a] The method according to [7] or [7-1], wherein the gene involved in IFN-γ secretion is TBX21 gene.
[8] The method according to [1], wherein the hematopoietic stem cell (HSC) and/or the hematopoietic progenitor cell (HPC) is a cell differentiated from an iPS cell (iPSC).
   [8-1] The method according to [6-1] or [7-1], wherein the (b1) or (b2) hematopoietic stem cell (HSC) or the hematopoietic progenitor cell (HPC) is a cell differentiated from an iPS cell (iPSC).
[9] The method according to [8] or [8-1], wherein the iPSC is a T cell-derived iPS cell (T-iPSC).
[10] The method according to [9], wherein the T cell is a CD4 single-positive T cell.
[11] A Th1-type CD4 single-positive T cell obtained by the method according to [1].
[12] A Th2-type CD4 single-positive T cell obtained by the method according to [1].
[13] A Th1-type CD4 single-positive T cell substantially defective in a factor involved in IL-4 secretion.
[14] A Th2-type CD4 single-positive T cell substantially defective in a factor involved in IFN-γ secretion.
[15] The CD4 single-positive T cell according to [13] or [14], wherein the Th1-type CD4 single-positive T cell or the Th2-type CD4 single-positive T cell is a cell differentiated from an iPS cell (iPSC).
[16] The CD4 single-positive T cell according to [15], wherein the iPSC is a T cell-derived iPS cell (T-iPSC).
[17] A method for producing a Th1-type or Th2-type CD4 single-positive T cell, comprising a step of inducing a CD4 single-positive T cell from an iPS cell in which a gene involved in IL-4 secretion or a gene involved in IFN-γ secretion is knocked out.
[18] A Hematopoietic stem cell (HSC) and/or hematopoietic progenitor cell (HPC) substantially defective in a factor involved in IL-4 secretion or a factor involved in IFN-γ secretion.
   [18a] The hematopoietic stem cell (HSC) and/or hematopoietic progenitor cell (HPC) according to [18] which is substantially defective in a factor involved in IL-4 secretion.
   [18b] The hematopoietic stem cell (HSC) and/or hematopoietic progenitor cell (HPC) according to [18] which is substantially defective in a factor involved in IFN-γ secretion.
[19] A method for controlling differentiation into a Th1-type or Th2-type CD4 single-positive T cell, comprising a step of rendering a hematopoietic stem cell (HSC) and/or a hematopoietic progenitor cell (HPC) substantially defective in a factor involved in IL-4 secretion or a factor involved in IFN-γ secretion.
[20] A pharmaceutical composition comprising the CD4 single-positive T cell according to [11], [12], [13] or [14] .
[21] A Th1-type CD4 single-positive T cell obtained by the method according to [6-1].
[22] A Th2-type CD4 single-positive T cell obtained by the method according to [7-1].

### Advantageous Effects of Invention

The ability to efficiently produce Th1-type CD4SP T cells and Th2-type CD4SP T cells according to the present invention greatly contributes to the practical use and application of immune cell therapy using helper T cells.

### BRIEF DESCRIPTION OF THE DRAWINGS

FIG. 1 shows the signal transduction pathways involved in Th1 and Th2 differentiation and the proteins intended for substantial defectiveness by knockout, etc. in the present invention (T-bet and IL-4). Partially modified Fig3 of Usui T. (2007). Jpn. J. Clin. Immunol., 30(6), 419-427;
FIG. 2 shows the sequence results of TBX21KO 654 (top) and the sequence results of IL4KO 656 (bottom) in Example 1;
FIG. 3 shows the culture conditions (medium components, oxygen concentration) of when the iPS cell lines (IL4KO and TBX21KO iPS cell lines) confirmed to be homozygous mutants of TBX21 and IL4, respectively, were differentiated into hematopoietic progenitor cells (HPCs) by the Embryonic Body method (EB method) in Example 2;

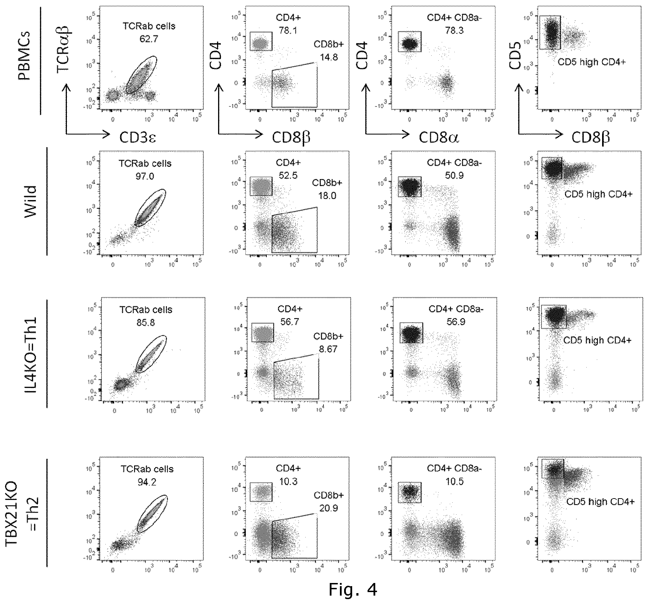

FIG. 4 shows the markers expressed in the cells obtained from ATOs prepared using HPCs derived from the IL4 gene knockout iPS cell line (IL4KO, the same applies hereafter), the TBX21 gene knockout iPS cell line (TBX21KO, the same applies hereafter), peripheral blood mononuclear cells (PBMCs, the same applies hereafter, may also be referred to as PBMC), and a wild-type iPS cell line (Wild, the same applies hereafter, may also be referred to as Wild-type) in which a predetermined gene has not been knocked out, in Example 2. In "IL4KO" and "TBX21KO", cells showing the expression pattern of "CD4+CD8β-" and the expression pattern of "CD4+CD8α-" similarly to "PBMCs" and "Wild" (within the frame line near each display), i.e., CD4SP T cells, are included;
FIG. 5 shows the chemokine receptor expression pattern of CD4SP T cells obtained from ATOs prepared using HPCs derived from each of IL4KO, TBX21KO, PBMC and Wild-type in Example 2;
FIG. 6 shows the cytokine secretion pattern of CD4SP T cells obtained from ATOs prepared using HPCs derived from each of IL4KO, TBX21KO, PBMC and Wild-type in Example 3; and
FIG. 7 shows the IL-13 production of various cells in Example 3. Left: Comparison between TKT3V1-7 wild-type cell-derived CD4SP T cells (TKT-Wild-CD4SPs) and primary CD4SP T cells (primary CD4SPs). Right: Comparison between TKT3V1-7 wild-type cell-derived CD4SP T cells (TKT-Wild-CD4SPs) and TKT3V1-7 IL4KO cell-derived CD4SP T cells (TKT IL4KO641/656-CD4SPs). Vertical axis: number of cells, horizontal axis: fluorescence intensity.

### DETAILED DESCRIPTION OF THE PREFERRED EMBODIMENTS

In the following description, "(B1) a hematopoietic stem cell (HSC) and/or a hematopoietic progenitor cell (HPC) substantially defective in a factor involved in IL-4 secretion" may be abbreviated as "HSPC (B1)", "(B2) a hematopoietic stem cell (HSC) and/or a hematopoietic progenitor cell (HPC) substantially defective in a factor involved in IFN-γ secretion" may be abbreviated as "HSPC (B2)", "(a) a stromal cell expressing a Notch ligand" may be abbreviated as "stromal cell(s) (a)", "(b1) a hematopoietic stem cell (HSC) or a hematopoietic progenitor cell (HPC), in which a gene involved in IL-4 secretion is knocked out, but a gene involved in IFN-γ secretion is not knocked out" may be abbreviated as "HSPC (b1)", and "(b2) a hematopoietic stem cell (HSC) or a hematopoietic progenitor cell (HPC), in which a gene involved in IFN-γ secretion is knocked out, but a gene involved in IL-4 secretion is not knocked out" may be abbreviated as "HSPC (b2)". The HSPC (b1) and the HSPC (b2) may be a HSC and a HPC in which a gene involved in IL-4 secretion is knocked out, but a gene involved in IFN-γ secretion is not knocked out, and a HSC and a HPC in which a gene involved in IFN-γ secretion is knocked out, but a gene involved in IL-4 secretion is not knocked out, respectively. In addition, "a hematopoietic stem cell and/or hematopoietic progenitor cell" (general term for a hematopoietic stem cell and a hematopoietic progenitor cell) may be abbreviated as "HSPC".

In the following description, an "IL-4 non-secreting and IFN-γ secreting (Th1-type) CD4 single-positive T cell" may be abbreviated as "Th1-type CD4SP T cell", and an "IFN-γ non-secreting and IL-4 secreting (Th2-type) CD4 single-positive T cell" may be abbreviated as "Th2-type CD4SP T cell". The Th1-type CD4SP T cell obtained by the method for producing the Th1-type CD4SP T cell of the present invention may be simply referred to as "Th1-type CD4SP T cell of the present invention", and the Th2-type CD4SP T cell obtained by the method for producing the Th2-type CD4SP T cell of the present invention may be simply referred to as "Th2-type CD4SP T cell of the present invention". In addition, "a CD4 single-positive T cell" may be abbreviated as "CD4SP cell". "CD4SP cells" in the Examples correspond to CD4 single-positive T cells.

In the present specification, a "hematopoietic stem cell" (HSC) is a multipotent stem cell that can differentiate into blood cells (leukocytes (neutrophils, eosinophils, basophils, lymphocytes, monocytes, macrophages), erythrocytes, platelets, mast cells and dendritic cells). Moreover, in the present specification, a "hematopoietic progenitor cell" (HPC) is a cell that has the ability to differentiate into blood cells but does not have the ability to self-renew as much as a stem cell. In humans, HSCs and HPCs are mainly present in bone marrow, but are also present in peripheral blood and cord blood, and can be collected from each site. In the present invention, the hematopoietic stem cell may be a cell isolated from living tissues such as bone marrow, blood, or the like, or may be a cell prepared from an ES cell or an iPS cell. Both hematopoietic stem cells (HSCs) and hematopoietic progenitor cells (HPCs) are cells that are CD34-positive and CD3-negative as cell markers (CD34+CD3-cells). Whether a cell is a hematopoietic stem cell can be confirmed by the fact that it survives when transplanted and grafted to an animal, removed again, and then transplanted to another individual, which means that it has the ability to self-renew, in other words, that it is a "stem cell", not a "progenitor cell".

In the present specification, the term an "induced pluripotent stem cell" (iPSC) refers to a cell obtained by introducing a specific factor (nuclear reprogramming factor) into a mammalian somatic cell or an undifferentiated stem cell to reprogram them. At present, there are various types of "induced pluripotent stem cells", and iPS cells established by introducing the four factors Oct3/4, Sox2, Klf4, and c-Myc into mouse fibroblasts by Yamanaka et al. (Takahashi K, Yamanaka S., Cell, (2006) 126: 663-676) can be used, as well as iPS cells derived from human cells established by introducing the same four factors into human fibroblasts (Takahashi K, Yamanaka S., et al. Cell, (2007) 131: 861-872), Nanog-iPS cells established by introducing the four factors, then selecting them by using the expression of Nanog as an index (Okita, K., Ichisaka, T., and Yamanaka, S. (2007). Nature 448, 313-317), iPS cells prepared by a method free of C-Myc (Nakagawa M, Yamanaka S., et al. Nature Biotechnology, (2008) 26, 101-106), and iPS cells established by introducing six factors by a virus-free method (Okita K et al. Nat. Methods 2011 May; 8(5): 409-12, Okita K et al. Stem Cells. 31(3): 458-66). In addition, the induced pluripotent stem cells established by introducing the four factors OCT3/4, SOX2, NANOG, and LIN28 prepared by Thomson et al. (Yu J., Thomson JA. et al., Science (2007) 318: 1917-1920), the induced pluripotent stem cells prepared by Daley et al. (Park IH, Daley GQ. et al., Nature (2007) 451: 141-146), the induced pluripotent stem cells prepared by Sakurada et al. (JP 2008-307007 A) and the like can also be used. Moreover, any of the induced pluripotent stem cells known in the art described in any published literature (for example, Shi Y., Ding S., et al., Cell Stem Cell, (2008) Vol3, Issue 5, 568-574; Kim JB., Scholer HR., et al., Nature, (2008) 454, 646-650; and Huangfu D., Melton, DA., et al., Nature Biotechnology, (2008) 26, No 7, 795-797), or patent (for example, JP 2008-307007 A, JP 2008-283972 A, US2008-2336610, US2009-047263, WO2007-069666, WO2008-118220, WO2008-124133, WO2008-151058, WO2009-006930, WO2009-006997, and WO2009-007852) can be used. As an induced pluripotent cell line, various iPS cell lines established by NIH, RIKEN, Kyoto University, and the like can be utilized. Examples of human iPS cell lines include RIKEN's HiPS-RIKEN-1A line, HiPS-RIKEN-2A line, HiPS-RIKEN-12A line, and Nips-B2 line, and Kyoto University's 253G1 line, 201B7 line, 409B2 line, 454E2 line, 606A1 line, 610B1 line, and 648A1 line. It should be noted that, in the present invention, a T cell-derived iPS cell line as described later can also be utilized.

In the present specification, the "marker" is a "marker protein" or "marker gene", and means a protein or gene thereof specifically expressed on the cell surface, in the cytoplasm, and/or nucleus of a predetermined cell type. The marker can be a positive selection marker or a negative selection marker. Preferably, the marker is a cell surface marker, and in particular, a cell surface positive selection marker allows the condensation, isolation, and/or detection of viable cells.

The detection of a marker protein can be performed by utilizing an immunological assay using an antibody specific to the marker protein, for example, ELISA, immunostaining, flow cytometry, and the like. As the antibody specific to the marker protein, an antibody that binds to a specific amino acid sequence in the marker protein, or to a specific sugar chain bound to the marker protein, can be used. In addition, techniques such as intracellular fluorescent staining using an antibody specific for the marker protein or expression of a reporter protein together with the marker protein can be used after fixing the cells, for marker proteins that are expressed inside the cell but do not appear on the cell surface (on the cell membrane), or are secreted from the cells (for example, transcription factors or their subunits, cytokines, and the like). It is preferable that this method be used when no suitable cell surface marker is found. On the other hand, the detection of a marker gene can be performed by utilizing a nucleic acid amplification method and/or a nucleic acid detection method known in the art, for example, RT-PCR (including quantitative PCR), microarray, biochip, RNAseq and the like.

In the present specification, a "positive" marker or the like means that the expression level (measured value or signal which reflects it) of the marker protein or gene exceeds (or is equal or more than) the detectable amount by a method known in the art as described above, or a predetermined reference value. In the present specification, a "negative" marker or the like means that the expression level of the marker protein or gene is below (or is equal or less than) the detectable amount by all or any of the methods known in the art as described above, or a predetermined reference value. The detectable amount or reference value of the protein or gene expression may vary depending on the method adopted and the purpose of analysis. For example, in the case of the expression level (secretion level) of a protein that serves as a marker, it can be determined as "positive" when the fluorescence signal in flow cytometry that stains cells with fluorescently labeled antibodies (typically, fluorescence-activated cell sorting: FACS) is higher (equal or more) than a predetermined standard set based on the fluorescence signal of unstained cells, and as "negative" when it is lower (equal or less). Positive expression may be indicated by a "+" symbol and negative expression may be indicated by a "-" symbol (for example, "CD4+CD8-" is synonymous with "CD4 positive CD8 negative").

"IFN-y" refers to "interferon y" (a protein mainly produced from antigen-stimulated Th1 and known to have various functions such as antiviral enhancement, antitumor action, and immune response regulation). "IL-4" refers to "interleukin 4" (a protein known to have various functions such as the differentiation and proliferation of antigen-stimulated naive CD4+ cells into Th2 cells, as well as B cell differentiation and antibody production, and regulation of M2 macrophage differentiation). "CXCR3" refers to a shared receptor specific for chemokines CXCL9 (also known as MIG: monokine induced by interferon γ) and CXCL10 (also known as IP-10; interferon-inducible protein 10), that is selectively expressed in Th1 cells. "CCR4" refers to a shared receptor specific for chemokines CCL17 (also known as TARC: thymus and activation-regulated chemokine) and CCL22 (also known as MDC: macropharge-derived chemokine), that is selectively expressed in Th2 cells.

In the present invention, "IL-4 non-secreting and IFN-γ secreting" means that the expression as an intracellular protein measured by, for example, flow cytometry (FACS, etc.) is "negative" for IL-4 and "positive" for IFN-γ. "IFN-γ non-secreting and IL-4 secreting" means that the expression as an intracellular protein measured by, for example, flow cytometry (FACS, etc.) is "positive" for IL-4 and "negative" for IFN-γ.

In the present specification, protein "secretion" is synonymous with "positive" and protein "non-secretion" is synonymous with "negative", unless otherwise stated.

In the present specification, a "stromal cell" is a cell constituting the connective tissue that supports parenchymal cells in a living tissue, and particularly refers to a stromal cell capable of producing an ATO that produces a T cell, typically, a stromal cell contained in hematopoietic tissues such as bone marrow or thymus. The stromal cell may be an established cell such as MS-5, OP9, and S17, which are mouse bone marrow cell lines, or HS-5, and HS-27a, which are human stromal cell lines, or may be a primary cell collected from humans or the like, or a cell differentiated from a pluripotent stem cell (iPS cell or the like) of humans or the like.

### - Production method -

The method for producing a Th1-type CD4 single-positive T cell (Th1-type CD4SP T cell) or a Th2-type CD4 single-positive T cell (Th2-type CD4SP T cell) of the present invention comprises a step of inducing a CD4 single-positive T cell (CD4SP T cell) from a hematopoietic stem cell and/or a hematopoietic progenitor cell (HSPC) substantially defective in a factor involved in IL-4 secretion or a factor involved in IFN-γ secretion. Specifically, the method for producing a Th1-type CD4SP T cell of the present invention comprises a step of inducing a CD4SP T cell from HSPC (B1) substantially defective in a factor involved in IL-4 secretion (on the other hand, not substantially defective in a factor involved in IFN-γ secretion). The method for producing a Th2-type CD4SP T cell of the present invention comprises a step of inducing a CD4SP T cell from HSPC (B2) substantially defective in a factor involved in IFN-γ secretion (on the other hand, not substantially defective in a factor involved in IL-4 secretion).

The "factor involved in IL-4 secretion" refers to a gene involved in IL-4 secretion, or a protein encoded by the gene. The "gene involved in IL-4 secretion" is intended for substantial defectiveness to prepare HSPC (B1) and not intended for substantial defectiveness to prepare HSPC (B2). The "gene involved in IL-4 secretion" is typically the "IL4 gene", but it may be another gene as long as it can suppress IL-4 secretion by substantial defectiveness and neither interferes with the production of a Th1-type CD4SP T cell nor suppresses IFN-γ secretion. More specifically, the "gene involved in IL-4 secretion" can be selected from genes encoding the proteins shown in the right figure of FIG. 1 (Th2 signals), that is, IL-4, IL-4R, Stat6, GATA3, mel-18, IL-5 and IL-13 (at least a portion of the protein if it is a complex). For example, if the GATA3 gene, which is known as a master regulator of Th2-type CD4SP T cells, or the IL4Ra (CD124) gene does not interfere with the differentiation into T cells by substantial defectiveness, and the suppression of IL-4 expression can be achieved, it can be used as a "gene involved in IL-4 secretion". HSPC (B1) can be similarly prepared even if a protein encoded by the gene is intended for substantial defectiveness, instead of the "gene involved in IL-4 secretion" as described above.

The "factor involved in IFN-γ secretion" refers to a gene involved in IFN-γ secretion, or a protein encoded by the gene. The "gene involved in IFN-γ secretion" is intended for substantial defectiveness to prepare HSPC (B2) and not intended for substantial defectiveness to prepare HSPC (B1). The "gene involved in IFN-γ secretion" is typically the "TBX21 gene" that encodes T-bet (see FIG. 1), but it may be another gene as long as it can suppress IFN-γ secretion by substantial defectiveness and neither interferes with the production of a Th2-type CD4SPT cell nor suppresses IL-4 secretion. More specifically, the "gene involved in IFN-γ secretion" can be selected from genes encoding the proteins shown in the left figure of FIG. 1 (Th1 signals), that is, IL-12, IFN-γ, IL-12Rβ2, IFNγR, Stat4, T-bet and HLX (at least a portion of the protein if it is a complex). For example, if the IFN-γ gene or the IL-12Rβ2 gene does not interfere with the differentiation into T cells by substantial defectiveness, it can be used as a "gene involved in IFN-γ secretion". HSPC (B2) can be similarly prepared even if a protein encoded by the gene is intended for substantial defectiveness, instead of the "gene involved in IFN-γ secretion" as described above.

The hematopoietic stem cell and/or the hematopoietic progenitor cell (HSPC) "substantially defective in" the predetermined factor mean HSPC in which signal transduction is not performed at the same level as in the case where the factor works normally. They are, for example, (i) HSPC in which the predetermined gene is modified so as not to express the predetermined protein, (ii) HSPC functionally defective in the predetermined (endogenous) protein, or (iii) HSPC having the reduced expression of the predetermined (endogenous) protein, and refer to those that exert the working effect of the present invention, that is, can induce the desired CD4SP T cells when cultured under appropriate conditions. Examples of the HSPCs (i) include HSPCs in which the gene is knocked out, and HSPCs in which at least one or some bases are added, substituted and/or deleted. Examples of the HSPCs (ii) include HSPCs in which a dominant negative mutant (for example, STAT6ΔC for STAT6 (FEBS letters 579 (2005) 3 953-3959)) is expressed and thereby works dominantly over the endogenous protein to attenuate the normal function of the endogenous protein. Examples of the HSPCs (iii) include HSPCs comprising shRNA and/or siRNA against the predetermined protein. Successful preparation of HSPCs "substantially defective in" the predetermined factor can also be confirmed by inducing CD4SP T cells from the HSPCs, and measuring IL-4 or IFN-γ secretion from the obtained CD4SP T cells. The "reduced expression of the predetermined protein" in (iii) means a decreased expression level of the predetermined protein in the HSPCs. In a certain embodiment, the expression level in HSPCs after treatment (for example, introduction of shRNA and/or siRNA against the predetermined (endogenous) protein to HSPCs) is reduced by at least about 25%, 40%, 50%, 65%, 70%, 75%, 80%, 85%, 90%, 95% or more (for example, 96%, 97%, 98% or 99%) as compared to the expression level before the treatment. In general, various means (methods, conditions, reagents, apparatuses, etc. for treatment) for preparing cells "substantially defective in" a certain gene or protein are known. A person skilled in the art can select an appropriate one from these means or design a means adaptable to the present invention, and thereby prepare HSPCs in which signal transduction is not performed at the same level as in the case where the predetermined factor (gene or protein) works normally, that is, HSPCs "substantially defective in" the predetermined factor, so as to exert the working effect of the present invention.

In this respect, one aspect of the present invention provides HSPC substantially defective in a factor involved in IL-4 secretion or a factor involved in IFN-γ secretion (HSPC substantially defective in a factor involved in IL-4 secretion, and HSPC substantially defective in a factor involved in IFN-γ secretion, respectively). Another aspect of the present invention provides a method for controlling differentiation into a Th1-type or Th2-type CD4 single-positive T cell, comprising a step of rendering a hematopoietic stem cell and/or a hematopoietic progenitor cell (HSPC) substantially defective in a factor involved in IL-4 secretion or a factor involved in IFN-γ secretion.

### <Knockout>

In a preferred embodiment of the present invention, the HSPC (B1) substantially defective in a factor involved in IL-4 secretion is HSPC (b1) in which a gene involved in IL-4 secretion is knocked out. In a preferred embodiment of the present invention, the HSPC (B2) substantially defective in a factor involved in IFN-γ secretion is HSPC (b2) in which a gene involved in IFN-γ secretion is knocked out.

HSPC (b1) and HSPC (b2) can be each produced by knocking out a predetermined gene in HSPC or in the original cell in which differentiation into HSPC is induced. For example, in the case of inducing differentiation of an iPSC to HSPC as described above, it is preferable to perform the knockout treatment at the iPSC stage.

The means for knocking out a predetermined gene is not limited, and various known means can be used. For example, the CRISPR/Cas system is one of the suitable means for knocking out a predetermined gene in the present invention. When the CRISPR/Cas system is applied to a nucleotide sequence contained in a predetermined gene locus on genomic DNA, double-strand breaks (DSB) of DNA occur repeatedly. As a result, repair errors are induced in the non-homologous end-joining (NHEJ) or homologous recombination (HR) pathway, and mutations such as nucleotide deletions and insertions are introduced, resulting in the knockout of the predetermined gene due to frameshift.

As the CRISPR/Cas system, in addition to the typical CRISPR/Cas9 system, various variations are known, and various systems can be used in the present invention as well. The CRISPR/Cas system includes Class 1 (Type I, Type III, and Type IV) involving a plurality of RNA-induced nucleases (Cas) and Class 2 (Type II, Type V, and Type VI) involving a single Cas. Examples of suitable CRISPR/Cas systems include the Class 2 Type II CRISPR/Cas9 system and the Class 2 Type V CRISPR-Cas12a/Cpf1.

In the CRISPR/Cas system, guide RNA (gRNA) and RNA-induced nucleases are used. However, various variations are known for each, and various embodiments can be used in the present invention as well.

gRNA is composed of crRNA and tracrRNA. The crRNA and tracrRNA may be each separate RNA strands forming a complex, or may be a single RNA strand linked via a spacer (single guide RNA: sgRNA or chimeric RNA). A person skilled in the art can appropriately design the nucleotide sequence to be targeted in order to knock out each predetermined gene in HSPCs (nucleotide sequence adjacent to the PAM sequence), that is, the crRNA nucleotide sequence corresponding to the nucleotide sequence. gRNA can be introduced into cells in the form of RNA, or in the form of DNA containing a nucleotide sequence capable of producing the RNA by transcription (such as an expression plasmid). gRNA in the form of RNA may consist only of natural nucleotides (adenine, guanine, cytosine, uracil), or may contain natural nucleotides and nucleotide analogs. Examples of nucleotide analogs include sugar-modified nucleotides (such as 2'-O-methylribose, 2'-O-propylribose, 2'-O-methoxyethoxyribose, 2'-O-methoxyethylribose, 2'-O-[2-(guanidium)ethyl]ribose, and 2'-O-fluororibose); bridged synthetic nucleic acids (BNA) (such as locked synthetic nucleic acids (LNA), and ethylene bridged synthetic nucleic acids (ENA)); and phosphodiester bond-modified nucleotides (such as the products of the substitution of the phosphodiester bond into a phosphorothioate bond, or into a N3'-P5' phosphoramidate bond). In one embodiment of the invention, a portion of the gRNA (preferably at least one nucleotide at each of the 3' and 5' end of the nucleotide sequence, more preferably two or three nucleotides each) can be a nucleotide analog (preferably a sugar-modified nucleotide and a phosphodiester bond-modified nucleotide, more specifically, 2'-O-methylribose and the product of the substitution of the phosphodiester bond into a phosphorothioate bond).

The RNA-induced nuclease may be a wild-type nuclease in which both of the two nuclease domains are active, or it may be a mutant nuclease (nickase) in which only one domain is active. The RNA-induced nuclease can be introduced into the cells in the form of a protein, or in the form of a nucleic acid (mRNA, or DNA such as an expression plasmid, and the like) that contains the nucleotide sequence encoding the amino acid sequence of the protein.

The means for introducing the predetermined RNA and RNA-induced nuclease to be used in the CRISPR/Cas system into the cells (in the present invention, for example, in the HSPCs) is not limited. In one embodiment of the present invention, the RNA strand of gRNA and the protein RNA-induced nuclease (such as Cas9) can be introduced into the cells by electroporation.

The various cells used in the present invention, that is, the HSPCs (B1) and (b1) and HSPCs (B2) and (b2), as well as the iPS cell for preparing HSPC (B1) etc. and HSPC (B2), etc. as described below, and further, the cell (a T cell and the like) for preparing the iPS cell may be derived from humans, or may be derived from animals other than humans, for example, mammals such as mice, rats, dogs, pigs, and monkeys, and can be selected according to the intended use of the Th1-type CD4SP T cell or the Th2-type CD4SP T cell obtained by the present invention. For example, when Th1-type CD4SP T cells or Th2-type CD4SP T cells are intended to be administered to humans, it is preferable that the various cells be derived from humans.

How the HSPC for preparing HSPC (B1) and HSPC (B2), etc., that is, a cell before being rendered substantially defective in the predetermined factor, is obtained or prepared is not limited. For example, it may be prepared by inducing differentiation from an iPS cell, an ES cell, or another cell capable of differentiating into HSPC, or it may be a HSC or a HPC collected from cord blood, bone marrow, peripheral blood, or other living body.

### <Induction of differentiation from iPSC to HSPC>

In a preferred embodiment of the present invention, HSPC is a cell differentiated from an iPS cell (iPSC). For example, a Th1-type CD4SP T cell and a Th2-type CD4SP T cell that are HLA compatible with the patient and do not cause immune rejection can be obtained by preparing an iPSC using a cell (preferably a T cell, more preferably a CD4SP T cell) collected from a patient scheduled to be administered the Th1-type CD4SP T cell and the Th2-type CD4SP T cell obtained by the production method of the present invention, and using the ATO prepared using the iPSC. Moreover, a universalized iPSC (having a specific HLA that does not cause immune rejection in a large number of patients, or having HLA knocked out) may also be used.

The basic matters and specific embodiments for inducing differentiation of iPSCs to HSPCs are known and are not limited. The medium composition, culture conditions, and the like can be appropriately adjusted and changed according to the differentiation induction method adopted, the number of culture days, and the like, and preferably so that the efficiency of differentiation induction to HSPCs and the cell growth rate is increased.

In a preferred embodiment of the present invention, the HSPC prepared from an iPSC (preferably a T-iPSC described below, the same applies hereinafter) are prepared by a feeder-free method. For example, the "Embryonic body method (EB method)" described in AE Grigoriadis et al. (2010). Blood, 115(14): 2769-2776, in which an embryonic body is formed to produce HPCs without using feeder cells, is preferable.

In addition, for example, iPSCs can be cultured on feeder cells to induce differentiation into HSPCs, as described in WO 2011/096482 and WO 2013/176197. It is preferable that the feeder cells be stromal cells from the viewpoint of facilitating the induction of differentiation into a mesoderm system. It is preferable that the stromal cells be OP9 cells, 10T1/2 cells (C3H10T1/2 cells) or the like that have been treated with irradiation or the like from the viewpoint of facilitating the induction of differentiation into a hematopoietic lineage.

Examples of the medium for a feeder-free method (such as the EB method) include StemFit (registered trademark, Ajinomoto Co., Inc.) and StemPro (registered trademark, Thermo Fisher Scientific). Examples of the medium for co-culturing iPSCs and feeder cells include X-VIVO medium, Iscove's Modified Dulbecco's Medium (IMDM), α-MEM, and DMEM. However, IMDM is preferable from the viewpoint of having a high efficiency of formation of bag-shaped structures (sacks) containing HSPCs and the like.

The medium may not necessarily contain cytokines. However, it is preferable that the medium contain cytokines and, if necessary, serum (for example, fetal bovine serum (FBS)), insulin, transferrin, sodium selenite, L-glutamine (or GlutaMAX (Gibco)), α-monothioglycerol, ascorbic acid and the like. It is preferable that the cytokine be at least one cytokine selected from the group consisting of BMP, bFGF, VEGF, SCF, TPO and FLT3L from the viewpoint of efficiently inducing differentiation of iPSCs into HSPCs. In the EB method, for example, it is preferable to use a combination of the three cytokines BMP4, bFGF and VEGF, a combination of the three cytokines bFGF, VEGF and SCF, or a combination of the five cytokines bFGF, VEGF, SCF, TPO and FLT3L, while changing according to the number of culture days. In the method of co-culturing iPSCs and feeder cells, for example, a combination of the three cytokines VEGF, SCF and TPO, or a combination of the three cytokines VEGF, SCF and FLT3L is more preferable.

The culturing period should be the period until which a sufficient number of HSPCs are produced (in the method of co-culturing iPSCs and feeder cells, until sacks containing them are formed), and is preferably 10 to 14 days from the start of culturing, for example. The culture environment is not limited, but is preferably about 5% CO₂ and about 37°C. In addition, from the viewpoint of increasing the production efficiency of HSPCs (efficiency of sack formation), it is more preferable to culture under the conditions of low oxygen concentration (for example, 5 to 20%) .

HSPCs and other cells (such as blood cells) are usually contained in the sacks formed by the differentiation induction as described above. The cells present inside the sacks can be separated and recovered from the sacks by physical means, for example, by passing them through a sterilized sieve-like tool (such as a cell strainer). Since HSPCs are cells expressing CD34 as a cell surface marker, they can be isolated from the cell population obtained by culturing, for example, by flow cytometry using fluorescently labeled anti-CD34 antibodies and a cell sorter. In one preferred embodiment of the present invention, HSPCs are HPCs.

### <Preparation of iPSC from T cell>

In a preferred embodiment of the present invention, the iPSC for inducing differentiation into HSPC is a T cell-derived iPSC (T-iPSC). The basic matters and specific embodiments for preparing an iPSC from a T cell are known, and for example, WO 2011/096482 and WO 2013/176197 can be referred to. The basic matters and specific embodiments for preparing an iPSC from a cell other than a T cell are also known.

The "T cell" for preparing an iPSC includes a cell expressing a T cell receptor (TCR) on the surface, particularly the α and β chains of TCR (TCRαβ), and a progenitor cell thereof, that is, a pro-T cell that does not express TCRαβ, a pre-T cell in which TCRβ and pre-TCRα are associated, and the like. In addition, the "T cell" includes a T cell in which both CD4 and CD8 expressions are negative (CD4/CD8 double negative T cell), a T cell in which both CD4 and CD8 expressions are positive (CD4/CD8DP T cell), a T cell with positive CD4 expression and negative CD8 expression (CD4SP T cell), and a T cell with positive CD8 expression and negative CD4 expression (CD8SP T cell). The explanation of "positive" or "negative" expression is as described separately in the present specification. In the present invention, particularly for the "CD4SP T cell" produced from an ATO, the "predetermined standard" (threshold value) for determining that CD4 is "positive" and CD8 is "negative" can be found by referring to the expression levels of CD4 and CD8 in a natural helper T cell.

As the T cells for preparing iPSCs, for example, T cells collected from tissues or the like containing T cells, such as peripheral blood, lymph node, bone marrow, thymus, spleen, cord blood, and affected tissues can be used.

In a preferred embodiment of the present invention, the T cell for preparing an iPSC is a CD4 single-positive T cell (CD4SP T cell). A CD4SP T cell can be isolated from a cell population contained in a tissue, for example, by flow cytometry using fluorescently labeled anti-CD4 antibodies and a cell sorter. iPS cell lines prepared from CD4SP T cells have been established, and for example, TKT3V1-7, b3a2 and the like can be used to prepare iPSCs in the present invention.

The collected T cell has antigen specificity, that is, an antigen-specific TCR provided by the reconstituted TCR gene. The Th1- or Th2-type CD4SP T cell of the present invention produced from an ATO prepared using an iPSC has an identical or substantially identical antigen specificity (antigen-specific TCR) to those possessed by the T cell used to prepare the iPSC. The T cell having the desired antigen specificity can be isolated from tissue by, for example, purification using an affinity column or the like on which the desired antigen is immobilized, or purification using MHC (major histocompatibility complex) multimers (for example, "MHC tetramers", "Pro 5 (registered trademark) MHC class I pentamers") and the like to which the desired antigen is bound.

In the production method of the present invention, a means (method, conditions, apparatus, etc. for culture) for inducing the desired CD4SP T cell, that is, Th1-type CD4SP T cell and Th2-type CD4SP T cell, from HSPC substantially defective in a factor involved in IL-4 secretion or a factor involved in IFN-γ secretion, that is, HSPC (B1) and (B2), respectively is not limited. A person skilled in the art can select an appropriate means or design culture conditions (medium, period, temperature, atmosphere, etc.) adaptable to the present invention, and thereby induce the CD4SP T cell by culturing the HSPC. A Th1-type CD4SP T cell and a Th2-type CD4SP T cell can be induced, for example, by culturing HSPC (B1) and (B2) in accordance with "ATO method". Hereinafter, ATO method targeting HSPCs (b1) and (b2) in which the predetermined gene is knocked out will be specifically described as a typical example of an induction method (culture method). However, the induction method (culture method) that can be used in the production method of the present invention is not limited thereto. In addition, HSPCs (B1) and (B2) other than HSPCs (b1) and (b2) may be used. The present invention can also be carried out according to an induction method (culture method) other than ATO method with reference to the following items described about the ATO method in a necessary scope (for example, regarding a medium).

### <ATO method>

In one preferred embodiment, the method for producing a Th1-type CD4SP T cell of the present invention comprises
a step of culturing an artificial thymic organoid (ATO) comprising:
   (a) a stromal cell expressing a Notch ligand; and
   (b1) a hematopoietic stem cell (HSC) and/or a hematopoietic progenitor cell (HPC) in which a gene involved in IL-4 secretion is knocked out, but a gene involved in IFN-γ secretion is not knocked out,
   thereby producing an IL-4 non-secreting and IFN-γ secreting (Th1-type) CD4SP T cell from the ATO.

In one preferred embodiment, the method for producing a Th2-type CD4SP T cell of the present invention comprises
a step of culturing an artificial thymic organoid (ATO) comprising:
   (a) a stromal cell expressing a Notch ligand; and
   (b2) a hematopoietic stem cell (HSC) and/or a hematopoietic progenitor cell (HPC) in which a gene involved in IFN-γ secretion is knocked out, but a gene involved in IL-4 secretion is not knocked out,
   thereby producing an IFN-γ non-secreting and IL-4secreting (Th2-type) CD4SP T cell from the ATO.

The ATO method in the production method of the present invention can be performed by preparing an ATO and collecting the produced cell by essentially the same manner as the conventional manner except that the HSPC (B1) or (B2), in which a specific factor has been substantially defective beforehand, preferably the HSPC (b1) or (b2), in which a specific gene has been knocked out beforehand, is used. The basic matters and specific embodiments of the ATO method is known (see, for example, Patent Literature 1: WO 2017/075389), and while the present invention is in accordance with these, some parts may be modified as necessary. As an example, in the method described in Patent Literature 1, an ATOs are prepared by forming embryonic mesodermal organoids on an insert from the beginning to obtain cell components corresponding to HPCs. However, this part may be modified and the differentiation from iPSCs to HPCs may be induced by the "Embryonic body method (EB method)" (AE Grigoriadis et al. (2010). Blood, 115(14): 2769-2776), then the ATOs may be prepared using the HPCs. In the present invention, the HSPCs used to prepare the ATOs are replaced with the HSPCs (b1) or (b2) in which a specific gene has been knocked out beforehand. In addition, in the present invention, the cells to be recovered among the cells produced by the ATOs are the Th1-type CD4SP T cells and Th2-type CD4SP T cells.

The medium for culturing ATOs (stromal cells (a) and HSPCs (b1) or (b2)) can be selected essentially from the media used for culturing animal cells, and can be used by adding the necessary components at an appropriate concentration. The medium and added components can be changed over the period of culture.

Examples of the medium include AIM V, X-VIVO-15, NeuroBasal, EGM2, TeSR, BME, BGJb, CMRL 1066, Glasgow MEM, improved MEM zinc option, IMDM, medium 199, Eagle's MEM, αMEM, DMEM, Ham, RPMI-1640 and Fisher medium. These media may be used alone, or two or more of them may be used in combination. An example of a preferred medium is RPMI-1640.

The medium may be a serum-containing medium, a serum-free medium, or a xeno-free medium. From the viewpoint of preventing contamination by heterologous animal-derived components, the serum may be derived from the same animal as the cells to be cultured. A serum-free medium refers to a medium that does not have unprocessed or unpurified serum, and thus may include media that have purified blood-derived components or animal tissue-derived components (such as growth factors). The medium may or may not contain any alternative to serum. Serum alternatives may include materials that appropriately contain albumin (such as lipid-rich albumin, bovine albumin, albumin substitutes such as recombinant albumin or humanized albumin, plant starch, dextran, and protein hydrolysates), transferrin (or other iron transporters), fatty acids, insulin, collagen precursors, trace elements, 2-mercaptoethanol, 3'-thioglycerol (α-monothioglycerol, MTG), or their equivalents. Commercially available materials such as Knockout Serum Replacement (KSR), Chemically-defined Lipid concentrated (Gibco), and GlutaMAX (Gibco) can also be used. The medium may be a serum-free medium (SFM) suitable for cell development. For example, the medium may contain a B-27 (registered trademark) supplement, Xeno-free B-27 (registered trademark) supplement, NS21 supplement, GS21 (registered trademark) supplement, or a combination thereof at a concentration effective for producing T cells from 3D cell aggregates.

The medium may contain one or more selected from the group consisting of biotin; DL-alpha-tocopherol acetate; DL-alpha-tocopherol; vitamins such as vitamin A (acetate); BSA (bovine serum albumin) or human albumin, fatty acid-free fraction V; catalase; human recombinant insulin; human transferrin; proteins such as superoxide dismutase; corticosterone; D-galactose; ethanolamine HCl; glutathione (reduced form); L-carnitine HCl; linoleic acid; linolenic acid; progesterone; putrescine 2HCl; sodium selenite; and T3 (triiodo-L-thyronine); PSG (penicillin, streptomycin, and L-glutamine). The medium may contain externally added ascorbic acid or a derivative thereof (for example, ascorbic acid 2-phosphate: PAA). The medium may contain one or more selected from the group consisting of fatty acids or lipids, amino acids (such as non-essential amino acids), vitamins, growth factors, cytokines, antibiotics, antioxidants, 2-mercaptoethanol, pyruvic acid, buffers, and inorganic salts, each added externally.

The medium may contain cytokines added externally. Examples of cytokines include FLT3 ligand (FLT3L), interleukin 7 (IL-7), stem cell factor (SCF), thrombopoietin (TPO), IL-2, IL-3, IL-4, IL-6, IL-12, IL-15, IL-21, TNF-alpha, TGF-beta, interferon-gamma, interferon-lambda, TSLP, thymopentin, pleotrophin, and midkine. These cytokines may be used alone, or two or more of them may be used in combination.

The 3D cell aggregate may or may not include an exogenous extracellular matrix or scaffold. Examples of exogenous extracellular matrices include collagen, gelatin, poly-L-lysine, poly-D-lysine, laminin, and fibronectin, as well as mixtures thereof, such as Matrigel (trademark), and lysed cell membrane preparations.

The other conditions for ATO culture can be set appropriately. The culture temperature can be, for example, 20 to 40°C, preferably about 37°C. The CO₂ concentration can be, for example, 2 to 10%, preferably 2 to 5%. The oxygen concentration can be, for example, 1 to 20%, preferably 5 to 20%.

The stromal cell (a) and HSPC (b1) or HSPC (b2) in the ATO can be co-cultured at any ratio. For example, the stromal cells (a): HSPCs (b1) or HSPCs (b2) can be 100:1 to 1:100, 90:1~1:90, 80:1~1:80, 70:1~1:70, 60:1~1:60, 50:1~1:50, 40:1~1:40, 30:1~1:30, 20:1~1:20, preferably 20:1 to 1:4. When HSPCs (b1) or HSPCs (b2) are prepared from T cell-derived iPS cells, the ratio is preferably about 1 (for example, MS5hDLL4):4 (for example, HPC). On the other hand, when HSPCs (b1) or HSPCs (b2) are prepared from non-T cell derived iPS cells, the ratio is preferably about 20 (for example, MS5hDLL4):1 (for example, HPC).

The culture period for the ATOs can be, for example, 4 weeks or more, preferably 5 weeks or more, more preferably 6 weeks or more, and further preferably 8 weeks or more or 9 weeks or more. The upper limit of the culture period is not limited, and can be preferably 16 weeks or less, more preferably 14 weeks or less, and further preferably 12 weeks or less. Specifically, the culture period can be, for example, 4 to 16 weeks, preferably 5 to 16 weeks, more preferably 6 to 14 weeks, and further preferably 8 to 12 weeks or 9 to 12 weeks. The longer the culture period for the ATOs is, about 4 to 9 weeks, the higher the proportion of CD4SP T cells in the obtained cell population tends to be. However, beyond a certain period, the proportion of CD4SP T cells increases less and the number of CCR7-positive cells may decrease (differentiation progresses toward the effector). Therefore, the culture period for ATOs can be adjusted within the above range depending on the cell line, culture conditions, and the like (for example, set to about 9 weeks), so as to obtain a cell population having the desired composition.

The cells produced from the ATOs in the production method of the present invention may include a CD8SP T cell, a CD4/CD8DN T cell and a CD4/CD8DP T cell in addition to a CD4SP T cell. For example, the CD4SP T cell can be isolated and recovered from the produced cells by flow cytometry (typically fluorescence-activated cell sorting: FACS) using fluorescently labeled anti-CD4 antibodies and a cell sorter. In addition, the presence of a Th1-type CD4SP T cell and a Th2-type CD4SP T cell among the CD4SP T cells can be confirmed by anti-IFN-y antibodies and anti-IL-4 antibodies each fluorescently labeled for intracellular staining, and flow cytometry. To recover a Th2-type CD4SP T cell as a living cell, it can be recovered, for example, as a CXCR3-negative, CCR4-positive (consistent with IL-4 secretion tendency), and CCR6 (a marker of Th17 cells) negative cell by flow cytometry.

### <Stromal cell (a)>

The stromal cell (a) can be prepared by introducing a Notch ligand gene into the stromal cell and expressing it. The stromal cell (a) may be derived from humans, or may be derived from animals other than humans, as in the HSPC, etc. described above. The means for introducing the Notch ligand gene is not limited, and a known appropriate means such as a viral vector (retroviral vector, lentiviral vector, and the like) can be adopted. For example, MS-5 mouse stromal cell lines transduced with human DLL1 and human DLL4 as Notch ligands (MS5-hDLL1 and MS5-hDLL4 lines, respectively) have been established, and such cell lines can be used as the stromal cells (a) of the present invention.

The term "Notch ligand" used in the present specification is synonymous with the term in Patent Literature 1: WO 2017/075389 (corresponding to JP 2018-533364 A) in which a method for producing ATOs and the like are described, and includes "canonical Notch ligands" and "non-canonical Notch ligands". Examples of canonical Notch ligands include Delta-like ligand 4 (DLL4), Delta-like ligand 1 (DLL1), Jagged1 (JAG1), Jagged2 (JAG2), Delta-like ligand 3 (DLL3), and X-delta 2. Examples of non-canonical Notch ligands include contactin-1, NOV/CCN3, contactin-6, Periostin/OSF-2, DLK2/EGFL9, Pref-1/DLK1/FA1, DNER, thrombospondin-2, MAGP-1/MFAP2, thrombospondin-3, MAGP-2/MFAP5, thrombospondin-4, and netrin-1.

### - Cells -

The first embodiment of the Th1-type CD4SP T cell of the present invention is a Th1-type CD4SP T cell obtained by the production method of the present invention described above, that is, the production method comprising a step of inducing a CD4 SPT cell from a hematopoietic stem cell and/or a hematopoietic progenitor cell (HSPC) substantially defective in a factor involved in IL-4 secretion. The second embodiment of the Th1-type CD4SP T cell of the present invention is a Th1-type CD4SP T cell substantially defective in a factor involved in IL-4 secretion. The Th1-type CD4SP T cell of the present invention may correspond to both of the first embodiment and the second embodiment. The Th1-type CD4SP T cell of the present invention is preferably, as described above, a cell differentiated from an iPS cell (iPSC), for example, a T cell-derived iPS cell (T-iPSC).

The first embodiment of the Th2-type CD4SP T cell of the present invention is a Th2-type CD4SP T cell obtained by the production method of the present invention described above, that is, the production method comprising a step of inducing a CD4 SPT cell from a hematopoietic stem cell and/or a hematopoietic progenitor cell (HSPC) substantially defective in a factor involved in IFN-γ secretion. The second embodiment of the Th2-type CD4SP T cell of the present invention is a Th2-type CD4SP T cell substantially defective in a factor involved in IFN-γ secretion. The Th2-type CD4SP T cell of the present invention may correspond to both of the first embodiment and the second embodiment. The Th2-type CD4SP T cell of the present invention is preferably, as described above, a cell differentiated from an iPS cell (iPSC), for example, a T cell-derived iPS cell (T-iPSC).

The first embodiment and the second embodiment of the Th1-type CD4SP T cell of the present invention can be distinguished from the natural Th1-type CD4SP T cells present in the living body (hereinafter, may be abbreviated as "natural Th1 cells") and a Th1-type CD4SP T cell obtained by a production method other than the present invention. For example, in one preferred embodiment of the production method of the present invention, a gene involved in IL-4 secretion in a hematopoietic stem cell and/or a hematopoietic progenitor cell (HSPC) is knocked out. In a Th1-type CD4SP T cell obtained by the production method, the gene involved in IL-4 secretion is also knocked out. Therefore, they can be clearly distinguished from the natural Th1 cells, etc. (in this case, the Th1-type CD4SP T cell correspond to both of the first embodiment and the second embodiment). It is possible to confirm that a predetermined gene has been knocked out by examining the nucleotide sequence of genomic DNA according to a conventional method. In addition, the second embodiment of the Th1-type CD4SP T cell of the present invention is substantially defective in a factor involved in IL-4 secretion and can therefore be distinguished from the natural Th1 cells, etc. that lack such a feature. Th1-type CD4SP T cells (first embodiment) obtained by other embodiments of the production method of the present invention can also be distinguished from the natural Th1 cells, etc. because of traces or the like according to features (particularly, a means of rending HSPCs substantially defective in a factor involved in IL-4 secretion) of the embodiments of the production method. Likewise, the first embodiment and the second embodiment of the Th2-type CD4SP T cell of the present invention can be distinguished from the natural Th2-type CD4SP T cells present in the living body (hereinafter, may be abbreviated as natural Th2 cells) and a Th2-type CD4SP T cell obtained by a production method other than the present invention.

The Th1-type CD4SP T cell and the Th2-type CD4SP T cell of the present invention are usually cells also expressing CD45RA or CD45RO, CD3ε, TCRαβ, and the like, in the same manner as natural Th1 cells and Th2 cells, respectively. In terms of chemokine receptor expression, the Th1-type CD4SP T cell of the present invention is CXCR3 positive and CCR4 negative like the natural Th1 cells, and the Th2-type CD4SP T cell of the present invention is CXCR3 negative and CCR4 positive like the natural Th2 cells. In addition, the Th1-type CD4SP T cell and the Th2-type CD4SP T cell of the present invention also strongly express CD5 (strongly positive), that is, they are also a mature Th1-type CD4SP T cell and a mature Th2-type CD4SP T cell.

In addition to the above characteristics, the Th1-type CD4SP T cell and the Th2-type CD4SP T cell of the present invention contain fractions expressing CD28 and CCR7, which are representative of the phenotype of central memory T cells, and have a high ability to self-replicate.

### <Applications>

The applications of the Th1-type CD4SP T cell and the Th2-type CD4SP T cell of the present invention are not limited, but for example, they can be used for the treatment or prevention of tumors, infectious diseases (for example, chronic infectious diseases), allergic diseases, autoimmune diseases and the like.

Therefore, in one aspect of the present invention, the Th1-type CD4SP T cell and the Th2-type CD4SP T cell obtained by the production method of the present invention, as well as a pharmaceutical composition containing these cells (cell preparation), to be used for the treatment or prevention of the diseases and the like described above, are provided.

In addition, in another aspect of the present invention, a method (immune cell therapy) for administering the Th1-type CD4SP T cell and the Th2-type CD4SP T cell obtained by the production method of the present invention, or a pharmaceutical composition containing these cells (cell preparation), is provided for the treatment or prevention of the diseases and the like described above.

The pharmaceutical composition (cell preparation) according to the above embodiment (hereinafter referred to as "the pharmaceutical composition (cell preparation) of the present invention") may contain either the Th1-type CD4SP T cell or the Th2-type CD4SP T cell, or it may contain both at an arbitrary mixing ratio.

The pharmaceutical composition (cell preparation) of the present invention can be formulated by a known pharmaceutical method according to the administration route and the like, and can be prepared, for example, as an injection (such as an intravenous injection or drip injection), a liquid preparation, a suspension, an emulsion, or the like. In such formulation, pharmacologically acceptable carriers (media) and additives, specifically sterilized water, saline, vegetable oils, solvents, bases, emulsifiers, suspending agents, surfactants, stabilizers, vehicles, preservatives, binders, diluents, isotonic agents, soothing agents, bulking agents, disintegrants, buffers, coating agents, lubricants, colorants, solubilizing agents and the like can be appropriately used in combination, as necessary. Furthermore, other active ingredients (such as other drugs and cells, for example, CAR-introduced CD8SP T cells), immunostimulators, and the like can also be contained along with the Th1-type CD4SP T cells and/or Th2-type CD4SP T cells, according to the purpose of treatment or prevention.

The method for administering the pharmaceutical composition (cell preparation) of the present invention is not limited, but is preferably a parenteral administration, for example, intravenous, intraperitoneal, subcutaneous or intramuscular administration, or a topical administration to the affected area, and more preferably an intravenous administration or a topical administration to the affected area. The dose of the pharmaceutical composition (cell preparation) of the present invention can be appropriately adjusted according to the age, body weight, symptoms, and health condition of the subject, the dosage form, the administration method and the like.

The product of the pharmaceutical composition (cell preparation) of the composition of the present invention may be labeled to be used for the treatment or prevention of diseases and the like. For example, the information that it is used for the treatment or prevention of diseases and the like can be described on the body, container, packaging, and the like of the product, or in the instructions, package insert, promotional material, other printed materials, and the like of the product.

As used in the present specification and claims, unless otherwise required by context, singular terms shall include pluralities and plural terms shall include the singular. Thus, singular articles (e.g., "a", "an", "the" and the like in case of English) should also be understood as encompassing the concept thereof in the plural form unless specifically noted otherwise.

### Examples

### [Example 1] Production of iPS cells (TBX21 KO cell line and IL4 KO cell line)

TKT3V1-7 provided by Kyoto University (CiRA) was used as iPS cells. Undifferentiated iPS cells passaged and maintained in AK03N medium (manufactured by Ajinomoto) and subconfluent in the wells were collected as single cells using TripLE Select (manufactured by Thermo Fisher Scientific). The collected cells were resuspended in Opti-MEM (manufactured by Thermo Fisher Scientific) so as to be 1.0 × 10⁶ cells/100 µL per cuvette.

A guide RNA was designed for the TBX21 gene encoding the transcription factor T-bet and the IL4 gene encoding the cytokine IL-4, and was formed using Alt-R CRISPR-Cas9 crRNA (manufactured by Integrated DNA Technologies) and Alt-R CRISPR-Cas9 tracrRNA (manufactured by the same company) containing this recognition sequence. This guide RNA suspension was reacted with Alt-R S.p. Cas9 Nuclease 3NLS (manufactured by the same company) to form an RNP complex.

The RNP complex suspension and 1.0 × 10⁶ iPS cells suspended in Opti-MEM were mixed so that the final concentration of the RNP complex was 1.6 µM, and electroporation was performed on this mixture using a NEPA21 electroporator (manufactured by Nepa Gene).

It should be noted that at the time of the electroporation, Puro (puromycin resistance gene) plasmids are electroporated at the same time and cultured in a puromycin-added medium to indirectly select Cas9 proteinintroduced cells. This utilizes the correlation between the introduction of Cas9 proteins into cells and the introduction of plasmids.

The electroporated iPS cells were seeded on a 10 cm dish coated with iMatrix-511 (manufactured by Matrixome), and the colonies generated after culturing for 12 days were picked up and subcultured. The cells derived from each of these colonies were cell-lined and stocked, and the knockout of the target gene and its mutation form were confirmed by sequencing.

The sequencing was performed on PCR products with primers designed to sandwich the target sequence of the guide RNA. An example of the sequencing results is shown in FIG. 2. Even with a knockout (KO) treatment with the same guide RNA, the mutations generated by the cell lines were different, and Table 1 shows a list of the cell lines for which the sequence and traits have been confirmed. It was later confirmed that the desired traits were equally expressed among these cell lines regardless of the form of mutation.

### [Table 1]

**Table 1: List of KO cell lines obtained**

| | Parent line | No | gRNA | KO form |
|---|---|---|---|---|
| IL4KO | TKT3VI-7 | 656 | gRNA4 ^{*1} | 28 bp del. |
| | | 641 | gRNA5^{*2} | Mix of multiple forms of mutation |
| | | 660 | | 4 bp del. |
| | | 661 | | 5 bp del. |
| TBX21KO | TKT3VI-7 | 654 | gRNA2^{*3} | 7 bp del. |
| | | 658 | | 18 bp del. |
| | | 667 | | 1 bp ins. |

| | | | | |
|---|---|---|---|---|
| *1 Nucleotide sequence in the IL4 gene targeted by gRNA4 (SEQ ID NO: 1) 5'-GTGTCCGTGGACAAAGTTGC-3' *2 Nucleotide sequence in the IL4 gene targeted by gRNA5 (SEQ ID NO: 2) 5'-CAAGTGCGATATCACCTTAC-3' *3 Nucleotide sequence in the TBX21 gene targeted by gRNA2 (SEQ ID NO: 3) 5'-GCGGTACCAGAGCGGCAAGT-3' | | | | |

### [Example 2] Production of Th1 cells and Th2 cells from iPS cells (TBX21KO cell line and IL4KO cell line) (iPSC -> HPC / HPC -> Th1 cell or Th2 cell)

An iPS cell line, which was confirmed to be a homozygous mutant of the target gene TBX21 or IL4 by sequencing, was differentiated into hematopoietic progenitor cells (HPCs) by the Embryonic Body method (EB method). The EB method is a method in which an embryonic body is formed to produce hematopoietic progenitor cells without using feeder cells, as shown in AE Grigoriadis et al. (2010). Blood, 115(14): 2769-2776. In the present Example, it is modified as shown in FIG. 3.

For TKT3V1-7 or its KO cell line, the day on which differentiation induction was started for undifferentiated cells was set as day 0, and the cells were used for the ATO preparation on day 9. The EB cluster contained in the culture solution was removed using a 40 µm cell strainer (manufactured by Fisher Scientific), and only the floating cells were collected. The collected floating cells are considered to contain a fraction corresponding to hematopoietic progenitor cells, and in the present Example, they are used in bulk as ATO material without sorting.

The Artificial Thymic Organoid method is an organoid culture method which originally differentiates primary hematopoietic stem cells into αβ-type T cells, as reported by CS Seet et al. (2017). Nat Methods. 14(5): 521-530. In this original method, CD34-positive CD3-negative cells are used after being sorted by a cell sorter, and it is considered that hematopoietic progenitor cells may be sorted.

In the present Example, the ATO differentiation culture method was started by mixing bulk iPS cell-derived hematopoietic progenitor cells collected without sorting with MS5hDLL4 cells so that the cell number ratio be 4:1, placing the suspension droplets on an insert (MILLICELL INSERT 30MM ORGANOTYPIC PTFE 0.4UM 50/PK manufactured by Merck (Millipore)) and then suspending them together with the insert in the medium. The medium was prepared by adding B-27^{™} Supplement (50×), serum free (manufactured by Thermo Fisher Scientific) for 25-fold dilution and PSG (penicillin, streptomycin, and L-glutamine) for 100-fold dilution, and similarly, GlutaMAX for 100-fold dilution, to RPMI-1640 (manufactured by Wako), then adding IL-7 (final concentration: 5 ng/mL), and similarly Flt3L (5 µg/mL) and PAA (50 mg/mL). The "MS5hDLL4 cells" are cells prepared by introducing the hDLL4 gene into the mouse bone marrow cell line "MS-5" with a lentiviral vector.

When a mixture of iPS cell-derived HPCs and MS5hDLL4 is cultured on the insert suspended in the present medium for 9 to 12 weeks, CD4 single-positive (SP) cells and CD8 single-positive (SP) cells are differentiated. Since intense IL-4 secretion was observed in the preceding CD4SP cells derived from iPSCs without KO and peripheral blood mononuclear cells ("Wild" and "PBMCs" in FIG. 4), and high T-bet expression was also observed, it was assumed that CD4SP and CD8SP differentiation, or T cell differentiation itself may be inhibited by IL4KO and TBX21KO, but as shown in FIG. 4, CD4SP differentiation was obtained from all KO cell lines.

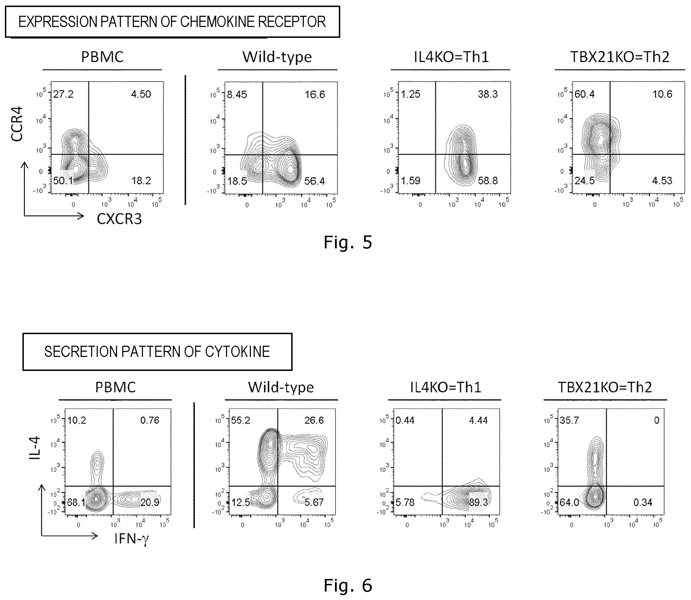

From the analysis of primary helper T cells, it is a common understanding that Th1 cells, which control cellmediated immunity, follow CXCR3-positive CCR4-negative, and Th2 cells, which control humoral immunity, follow CXCR3-negative CCR4-positive as chemokine receptor expression patterns. It was assumed that in IL4KO, which eliminates the secretion of IL-4, a functional cytokine of Th2, the cells show a Th1-type expression pattern, and that in the KO of TBX21, which encodes T-bet, a Th1 master regulator (a transcription factor that mainly induces differentiation), the cells show a Th2-type expression pattern. This was verified by analysis with a flow cytometer using anti-CXCR3 antibodies (clone: 1C6/CXCR3, manufactured by BD) and anti-CCR4 antibodies (clone: 1G1, manufactured by BD). As a result, as shown in FIG. 5, the CD4SP differentiated from the wild-type without KO treatment contained both CXCR3-positive CCR4-negative cells and CXCR3-negative CCR4-positive cells in the same manner as primary CD4SP cells in PBMC. By contrast, the CD4SP cells differentiated from IL4KO cells comprised only CXCR3-positive CCR4-negative cells, which was consistent with the expression pattern of Th1 cells. In addition, the CD4SP cells differentiated from TBX21KO cells comprised only CXCR3-negative cells, of which many were CCR4-positive. This was consistent with the expression pattern of Th2 cells.

### [Example 3] Confirmation of IL-4 and IFN-γ secretion of the obtained Th1 cells and Th2 cells

Next, the ability to produce cytokines of the CD4SP cells differentiated from IL4KO cells and of CD4SP cells differentiated from TBX21KO cells was evaluated by a technique of intracellular staining flow cytometry using anti-IFN-y antibodies (clone: B27, manufactured by BD) and anti-IL-4 antibodies (clone: 8D4-8, manufactured by Biolegend). As a result of stimulation with PMA (40 ng/mL) and ionomycin (4 µg/mL) for 3 hours under monensin (2 µM) addition, 55.2% of the CD4SP cells derived from wild-type iPS cells produced IL-4 and 5.67% produced IFN-γ, as shown in FIG. 6. 26.6% produced both. By contrast, 89.3% of the CD4SP cells differentiated from IL4KO cells secreted only IFN-γ. This was consistent with the behavior of Th1 cells. In addition, 35.7% of the CD4SP cells derived from TBX21KO cells secreted only IL-4, which is consistent with the behavior of Th2 cells, while 0.34% of the cells secreted only IFN-γ, and 0% of the cells secreted both. Thus, the cells secreting IFN-γ were almost eliminated. It was therefore confirmed that Th1 cells secreting only IFN-γ are obtained by IL4KO, and Th2 cells secreting only IL-4 are obtained by TBX21KO.

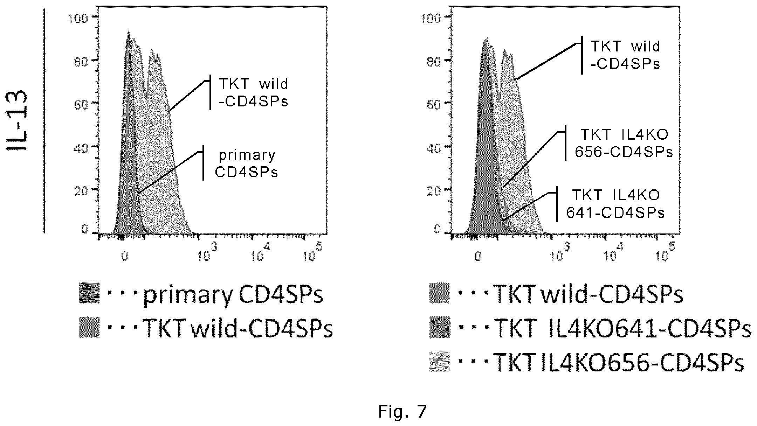

For the CD4SP cells derived from IL4KO cells, it was possible that it was only the elimination of IL-4 production due to the KO of the gene for the cytokine IL-4, which is the final product of Th2 cells, that was being observed. However, as seen in Example 2, the chemokine receptor expression pattern was also changed to that of Th1 cells, which is CXCR3-positive CCR4-negative, by IL4KO. Furthermore, the production of IL-13, which is a typical functional cytokine of Th2 cells along with IL-4, was verified by a technique of intracellular staining flow cytometry using anti-IL-13 antibodies (clone: JES10-5A2, manufactured by Biolegend). As a result, as shown in FIG. 7, while the CD4SP cells derived from wild-type iPS cells contained a fraction that highly produced IL-13 compared to primary CD4SP cells, the fraction highly secreting IL-13 had disappeared in the CD4SP cells derived from a plurality of IL4KO cell lines. The secretion of IL-13, whose gene was not directly knocked out by IL4KO, was reduced, which is one evidence that IL4KO interfered with the phenotypic expression of Th2 cells.

### SEQUENCE LISTING

See the attached sheets.

## Claims

1. A method for producing a Th1-type or Th2-type CD4 single-positive T cell, comprising
a step of inducing a CD4 single-positive T cell from a hematopoietic stem cell (HSC) and/or a hematopoietic progenitor cell (HPC) substantially defective in
a factor involved in IL-4 secretion, or
a factor involved in IFN-γ secretion.

2. The method according to claim 1, which is a method for producing a Th1-type CD4 single-positive T cell, comprising
a step of inducing a CD4 single-positive T cell from (B1) a hematopoietic stem cell (HSC) and/or a hematopoietic progenitor cell (HPC) substantially defective in a factor involved in IL-4 secretion.

3. The method according to claim 1, which is a method for producing a Th2-type CD4 single-positive T cell, comprising
a step of inducing a CD4 single-positive T cell from (B2) a hematopoietic stem cell (HSC) and/or a hematopoietic progenitor cell (HPC) substantially defective in a factor involved in IFN-γ secretion.

4. The method according to claim 1, wherein the HSC and/or HPC is (b1) a HSC and/or a HPC in which a gene involved in IL-4 secretion is knocked out.

5. The method according to claim 1, wherein the HSC and/or HPC is (b2) a HSC and/or a HPC in which a gene involved in IFN-γ secretion is knocked out.

6. The method according to claim 1, which is a method for producing a Th1-type CD4 single-positive T cell, comprising
a step of culturing an artificial thymic organoid (ATO) comprising:
(a) a stromal cell expressing a Notch ligand; and
(b1) a hematopoietic stem cell (HSC) and/or a hematopoietic progenitor cell (HPC) in which a gene involved in IL-4 secretion is knocked out, but a gene involved in IFN-γ secretion is not knocked out,
and thereby producing an IL-4 non-secreting and IFN-γ secreting (Th1-type) CD4 single-positive T cell from the ATO.

7. The method according to claim 1, which is a method for producing a Th2-type CD4 single-positive T cell, comprising
a step of culturing an artificial thymic organoid (ATO) comprising:
(a) a stromal cell expressing a Notch ligand; and
(b2) a hematopoietic stem cell (HSC) and/or a hematopoietic progenitor cell (HPC) in which a gene involved in IFN-γ secretion is knocked out, but a gene involved in IL-4 secretion is not knocked out,
and thereby producing an IFN-γ non-secreting and IL-4 secreting (Th2-type) CD4 single-positive T cell from the ATO.

8. The method according to claim 1, wherein the hematopoietic stem cell (HSC) and/or the hematopoietic progenitor cell (HPC) is a cell differentiated from an iPS cell (iPSC).

9. The method according to claim 8, wherein the iPSC is a T cell-derived iPS cell (T-iPSCs).

10. The method according to claim 9, wherein the T cell is a CD4 single-positive T cell.

11. A Th1-type CD4 single-positive T cell obtained by the method according to claim 1.

12. A Th2-type CD4 single-positive T cell obtained by the method according to claim 1.

13. A Th1-type CD4 single-positive T cell substantially defective in a factor involved in IL-4 secretion.

14. A Th2-type CD4 single-positive T cell substantially defective in a factor involved in IFN-γ secretion.

15. The CD4 single-positive T cell according to claim 13 or 14, wherein the Th1-type CD4 single-positive T cell or the Th2-type CD4 single-positive T cell is a cell differentiated from an iPS cell (iPSC).

16. The CD4 single-positive T cell according to claim 15, wherein the iPSC is a T cell-derived iPS cell (T-iPSC).
